# EUROPÄISCHE PATENTANMELDUNG

(11) **EP 2 017 256 A1**
(43) Veröffentlichungstag der Anmeldung: **21.01.2009**
(21) Anmeldenummer: 07112758.3
(22) Anmeldetag: 19.07.2007
(51) Int. Cl.: C07C 67/56

(54) **Verfahren zum Entfernen von Säuren aus (Meth)Acrylsäureestern durch filtrierende Adsorption an einem pulverförmigen Adsorptionsmittel**

(71) Anmelder: BASF SE, 67056 Ludwigshafen (DE)
(72) Erfinder: Lipowsky, Gunter, 69198, Schriesheim (DE); Hoefer, Frank, 67098, Bad Dürkheim (DE)
(74) Vertreter: Fitzner, Uwe

(57) **Zusammenfassung**

Verfahren zum Entfernen von Säuren aus (Meth)Acrylsäureestern, bei dem die Säuren durch filtrierende Adsorption an mindestens einem pulverförmigen Adsorptionsmittel von den (Meth)Acrylsäureestern abgetrennt werden; sowie die Verwendung von pulverförmigen Materialien, die mindestens einen Stoff, ausgewählt aus der Gruppe, bestehend aus organischen basischen Anionenaustauschern, polymeren aminogruppenhaltigen Basen, Molekularsieben, anorganischen basischen Salzen, Metalloxiden und Metallhydroxiden, enthalten oder hieraus bestehen, als filtrierende Adsorptionsmittel zur Entfernung von Säuren aus (Meth)Acrylsäureestern.

## Beschreibung

### Gebiet der Erfindung

Die vorliegende Erfindung betrifft ein neues Verfahren zum Entfernen von Säuren aus (Meth)Acrylsäureestern durch filtrierende Adsorption an mindestens einem pulverförmigen Adsorptionsmittel. Außerdem betrifft die vorliegende Erfindung die Verwendung von pulverförmigen Materialien, die mindestens einen Stoff, ausgewählt aus der Gruppe, bestehend aus organischen basischen Anionenaustauschern, polymeren aminogruppenhaltigen Basen, Molekularsieben, anorganischen basischen Salzen, Metalloxiden und Metallhydroxiden, enthalten oder hieraus bestehen, als filtrierende Adsorptionsmittel zur Entfernung von Säuren aus (Meth)Acrylsäureestern.

### Stand der Technik

Im Rahmen der vorliegenden Erfindung ist unter dem Ausdruck »(Meth)Acrylsäure« die Kurzfassung von »Acrylsäure oder Methacrylsäure« sowie »Acrylsäure und Methacrylsäure« zu verstehen. Demgemäß ist unter dem Ausdruck »(Meth)Acrylsäureester« die Kurzfassung von »Acrylsäureester oder Methacrylsäureester« sowie »Acrylsäureester und Methacrylsäureester« zu verstehen.

(Meth)Acrylsäureester, die durch die Veresterung von Acrylsäure und/oder Methacrylsäure mit aliphatischen Monoalkoholen mit 1 bis 8 Kohlenstoffatomen im Alkylrest, cycloaliphatischen Monoalkoholen mit 4 bis 7 Kohlenstoffatomen im Cycloalkylrest oder Olefinen wie Isobuten hergestellt werden, können noch beträchtliche Menge an Säuren wie (Meth)Acrylsäure, Diacrylsäure, Ameisensäure, Essigsäure und Propionsäure enthalten. Diese Säuren können die Rückspaltung der (Meth)Acrylsäureester bewirken. Man ist daher bestrebt, den Säuregehalt der (Meth)Acrylsäureester so weit wie möglich zu erniedrigen.

So ist es aus dem tschechischen Patent CZ 279 181 bekannt, den Methacrylsäuregehalt von Methylmethacrylat durch Destillation zu erniedrigen. Allerdings kann es bei der für die Destillation notwendigen thermischen Belastung zur unerwünschten Rückspaltung des Methylmethacrylats und/oder zur unerwünschten Oligomerisation und/oder Polymerisation der Methacrylsäure und des Methylmethacrylats kommen.

Die Adsorption oder die filtrierende Adsorption (vgl. Römpp Online 2007, »Filtrierende Adsorption«) von organischen Säuren an Feststoffen ist an sich bekannt. Im Rahmen der vorliegenden Erfindung umfasst der Begriff »filtrierende Adsorption« auch die Adsorption von Säuren aus lösemittelfreien (Meth)Acrylsäureestern.

So wird in der internationalen Patentanmeldung WO 2004/103516 A1 die Abtrennung von Säuren wie Schwefelsäure, Salzsäure oder para-Toluolsulfonsäure durch Adsorption an Polyiminen beschrieben.

Die Entfernung von Essigsäure aus Wasser durch Aktivkohle oder Ionenaustauscher geht aus dem Artikel von C. L. Munson, A. A. Garcia, Y. Kuo, M. Frierman, und C. J. King in Separation and Purification Methods, 1987, Band 16, Seiten 65 bis 89, hervor.

Nach dem Artikel von Y.-Y. Chen in Wuli Huaxue Xuebao, 2002, Band 18, Seiten 62 bis 65, können Propionsäure und Buttersäure aus Wasser durch Adsorption an Kieselgel entfernt werden.

Die Adsorption von Propionsäure aus Ethanol oder Benzol an unterschiedlich behandelten Glasoberflächen wurde untersucht. Aus Ethanol wurde keine Adsorption festgestellt. Aus Benzol war die Adsorption möglich, sie war aber unabhängig von der Oberflächenbehandlung (vgl. die Artikel von S. Furuuchi, H. Sakata und S. Noguchi in Asahi Garasu Kenyuku Hokoku, 1975, Band 25, Seiten 21 bis 28 und International Congress on Glass [Papers], 10th (1974), Meeting Date 1974, Band 9, Seiten 77 bis 84).

Natrium-n-tetradecylsulfat und Propionsäure wurden an Aktivkohle absorbiert, und die Diffusionsmechanismen wurden untersucht (vgl. den Artikel von M. J. Schwuger in Fortschrittsberichte über Kolloide und Polymere, 1971, Band 55, Seiten 91 bis 96).

Essigsäure und Propionsäure sowie deren Natrium-und Kaliumsalze wurden aus Wasser durch Adsorption an Aktivkohle entfernt (vgl. den Artikel von E. Angelescu und V. N. Comanescu in Bulletin de la Societé Chimique, Romania, 1928, Band 10, Seiten 170 bis 182).

Die Verwendung der Adsorption beziehungsweise der filtrierenden Adsorption zur Entfernung von Säuren aus (Meth)Acrylsäureestern ist nicht bekannt.

### Aufgabe der vorliegenden Erfindung

Der vorliegenden Erfindung lag die Aufgabe zu Grunde, ein neues Verfahren zur Entfernung von Säuren aus (Meth)Acrylsäureestern zu finden, das die Nachteile des Standes der Technik nicht mehr länger aufweist, sondern das es gestattet, den Säuregehalt von (Meth)Acrylsäureestern auf < 100 ppm zu senken, ohne dass es dabei zu einer nennenswerten Rückspaltung der (Meth)Acrylsäureester und/oder zu einer nennenswerten Oligomerisation und/oder Polymerisation der (Meth)Acrylsäureester und/oder der (Meth)Acrylsäure kommt.

Im Rahmen der vorliegenden Erfindung weist der Begriff »nennenswert« darauf hin, dass die Mengen der durch Rückspaltung und/oder Oligomerisation und/oder Polymerisation erzeugten unerwünschten Produkte entweder unter den Nachweisgrenzen der üblichen und bekannten Methoden für ihren Nachweis liegen oder dass die Mengen so gering sein müssen, dass sie weder die Durchführung des erfindungsgemäßen Verfahrens stören noch die Produktqualität vermindern.

Das neue Verfahren zur Entfernung von Säuren aus (Meth)Acrylsäureestern soll in einfacher Weise ohne großen zusätzlichen apparativen und sicherheitstechnischen Aufwand sowie Mess- und Regelaufwand durchführbar sein.

Außerdem war es die Aufgabe der vorliegenden Erfindung, eine neue Verwendung für pulverförmige Materialien, die mindestens einen Stoff, ausgewählt aus der Gruppe, bestehend aus organischen basischen Anionenaustauschern, polymeren aminogruppenhaltigen Basen, Molekularsieben, anorganischen basischen Salzen, Metalloxiden und Metallhydroxiden, enthalten oder hieraus bestehen, zu finden.

Nicht zuletzt sollten sich die mit Hilfe des erfindungsgemäßen Verfahrens erzeugten (Meth)Acrylsäureester eines Säuregehalts < 100 ppm hervorragend für die Herstellung von (Meth)Acrylat(co)polymerisaten für spezielle Anwendungszwecke wie Bindemittel für hochwertige Autoreparaturlacke eignen.

### Erfindungsgemäße Lösung

Demgemäß wurde das neue Verfahren zum Entfernen von Säuren aus (Meth)Acrylsäureestern gefunden, bei dem die Säuren durch filtrierende Adsorption an mindestens einem pulverförmigen Adsorptionsmittel von den (Meth)Acrylsäureestern abgetrennt werden und das im Folgenden als »erfindungsgemäßes Verfahren« bezeichnet wird.

Außerdem wurde die neue Verwendung von pulverförmigen Materialien, die mindestens einen Stoff, ausgewählt aus der Gruppe, bestehend aus organischen basischen Anionenaustauschern, polymeren aminogruppenhaltigen Basen, Molekularsieben, anorganischen basischen Salzen, Metalloxiden und Metallhydroxiden, enthalten oder hieraus bestehen, als filtrierende Adsorptionsmittel zur Entfernung von Säuren aus (Meth)Acrylsäureestern gefunden, was im Folgenden als »erfindungsgemäße Verwendung« bezeichnet wird.

### Vorteile der Erfindung

Im Hinblick auf den Stand der Technik war es überraschend und für den Fachmann nicht vorhersehbar, dass die Aufgabe, die der vorliegenden Erfindung zugrunde lag, mit Hilfe des erfindungsgemäßen Verfahrens und der erfindungsgemäßen Verwendung gelöst werden konnte.

Insbesondere war es überraschend, dass das erfindungsgemäße Verfahren auch im großtechnischen Maßstab rasch und in einfacher Weise ohne großen zusätzlichen apparativen und sicherheitstechnischen Aufwand sowie Mess- und Regelaufwand durchgeführt werden konnte.

Noch mehr überraschte, dass es aufgrund der erfindungsgemäßen Verwendung und des erfindungsgemäßen Verfahrens möglich war, den Säuregehalt von (Meth)Acrylsäureestern auf < 100 ppm, insbesondere < 50 ppm, zu senken, ohne dass es dabei zu einer nennenswerten Rückspaltung der (Meth)Acrylsäureester und/oder zu einer nennenswerten Oligomerisation und/oder Polymerisation der (Meth)Acrylsäureester und/oder der (Meth)Acrylsäure kam.

Nicht zuletzt eigneten sich die mit Hilfe des erfindungsgemäßen Verfahrens erzeugten (Meth)Acrylsäureester eines Säuregehalts < 100 ppm hervorragend für die Herstellung von (Meth)Acrylat(co)polymerisaten für spezielle Anwendungszwecke wie hochwertige Bindemittel für Autoreparaturlacke.

### Ausführliche Beschreibung der Erfindung

Das erfindungsgemäße Verfahren dient dem Entfernen von Säuren, vorzugsweise von organischen Säuren, aus (Meth)Acrylsäureestern.

Bekanntermaßen enthalten (Meth)Acrylsäureester, wie sie bei der großtechnischen Produktion anfallen, erhebliche Mengen an Säuren, wie nicht umgesetzte (Meth)Acrylsäure, Diacrylsäure und/oder gesättigte organische Säuren. Als gesättigte organische Säuren finden sich häufig Alkanmonocarbonsäuren mit 1 bis 4 Kohlenstoffatomen im Alkylrest, insbesondere Ameisensäure, Essigsäure und Propionsäure.

Außerdem können noch Reste von Säuren wie Schwefelsäure oder Sulfonsäuren, die üblicherweise als Veresterungskatalysatoren eingesetzt werden, vorhanden sein.

Vorzugsweise wird das erfindungsgemäße Verfahren mit (Meth)Acrylsäureestern durchgeführt, die durch säurekatalysierte Veresterung von (Meth)Acrylsäure hergestellt werden.

Bevorzugt wird die Veresterung mit mindestens einer Verbindung durchgeführt, die aus der Gruppe bestehend aus,
- aliphatischen Monoalkoholen vorzugsweise mit 1 bis 20, bevorzugt mit 1 bis 16 und besonders bevorzugt mit 1 bis 10 Kohlenstoffatomen im Alkylrest, insbesondere Methanol, Ethanol, Propanol, Isopropanol, n-Butanol, Isobutanol, tert.-Butanol, n-Pentanol, Isoamylalkohol, n-Hexanol, n-Heptanol, n-Octanol, n-Nonanol und n-Decanol;
- cycloaliphatischen Monoalkoholen vorzugsweise mit 4 bis 20, bevorzugt mit 5 bis 16 und besonders bevorzugt mit 6 bis 10 Kohlenstoffatomen im Cycloakylrest, insbesondere Cyclohexanol oder Monoalkohole, die sich von Norbornan, Bicyclo[2.2.2]octan, Decalin, Hydrindan oder Adamantan ableiten;
- aliphatisch-cycloaliphatischen Monoalkoholen, worin die Hydroxylgruppe mit einem aliphatischen oder einem cycloaliphatischen Kohlenstoffatom verknüpft ist, vorzugsweise mit 5 bis 30, bevorzugt mit 7 bis 20 und besonders bevorzugt mit 7 bis 10 Kohlenstoffatomen im Alkyl-Cycloakyl-Rest, insbesondere 2-, 3- oder 4-Methyl-, -Ethyl-, -Propyl-, -Isopropyl-, -n-Butyl- oder -tert.-Butyl-cyclohexanol und Hydroxymethyl-, 2-Hydroxyethyl-, 3-Hydroxypropyl- oder 4-Hydroxybutylcyclohexan;
- aliphatisch-aromatischen Monoalkoholen, worin die Hydroxylgruppe mit einem aliphatischen Kohlenstoffatom verknüpft ist, vorzugsweise mit 7 bis 30, bevorzugt mit 7 bis 20 und besonders bevorzugt mit 7 bis 10 Kohlenstoffatomen im Alkyl-Aryl-Rest, insbesondere Benzylalkohol, 2-Hydroxyethyl-, 3-Hydroxypropyl- oder 4-Hydroxybutylbenzol;
- cycloaliphatisch-aromatischen Monoalkoholen, worin die Hydroxylgruppe mit einem cycloaliphatischen Kohlenstoffatom verknüpft ist, vorzugsweise mit 10 bis 30, bevorzugt mit 10 bis 20 und besonders bevorzugt mit 10 bis 16 Kohlenstoffatomen im Cycloalkyl-Aryl-Rest, insbesondere 2-, 3- oder 4-Phenylcyclohexanol;
- aliphatisch-cycloaliphatischen-aromatischen Monoalkoholen, worin die Hydroxylgruppe mit einem aliphatischen oder einem cycloaliphatischen Kohlenstoffatom verknüpft ist, vorzugsweise mit 11 bis 30, bevorzugt mit 13 bis 20 und besonders bevorzugt mit 13 bis 16 Kohlenstoffatomen im Alkyl-Cycloalkyl-Aryl-Rest, insbesondere 1-Hydroxymethyl-2-, -3- oder -4-phenylcyclohexan, 2-Methyl-, -3-, -4-, -5- oder -6-phenylcyclohexanol oder 2-, 3- oder 4-Phenylbenzylalkohol; sowie
- Olefinen der allgemeinen Formel I: 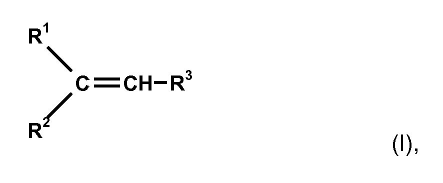 ausgewählt werden.

In der allgemeinen Formel I sind R¹ und R² gleich oder voneinander verschieden und stehen für einen Alkyl-Rest, vorzugsweise mit 1 bis 10, bevorzugt mit 1 bis 6 und besonders bevorzugt mit 1 bis 4 Kohlenstoffatomen, insbesondere aber für Methyl oder Ethyl.

In der allgemeinen Formel I steht R³ für
- ein Wasserstoffatom;
- einen Alkylrest vorzugsweise mit 1 bis 20, bevorzugt mit 1 bis 10 und besonders bevorzugt mit 1 bis 4 Kohlenstoffatomen, ganz besonders bevorzugt Methyl, Ethyl, Propyl, Isopropyl, n-Butyl, Isobutyl oder tert.-Butyl, insbesondere Methyl oder Ethyl;
- einen Cycloalkyrest vorzugsweise mit 4 bis 20, bevorzugt mit 5 bis 16 und besonders bevorzugt mit 6 bis 10 Kohlenstoffatomen, insbesondere Cyclohexan oder einen Rest, der sich von Norbornan, Bicyclo[2.2.2]octan, Decalin, Hydrindan oder Adamantan ableitet;
- einen Arylrest vorzugsweise mit 6 bis 30, bevorzugt mit 6 bis 20 und besonders bevorzugt mit 6 bis 14 Kohlenstoffatomen, insbesondere Phenyl oder einen Rest, der sich von Naphthalin und Phenanthren ableitet;
- einen Alkyl-Cycloalkyl-Rest, der über ein aliphatisches oder ein cycloaliphatisches Kohlenstoffatom mit dem olefinisch ungesättigten Kohlenstoffatom verknüpft ist, vorzugsweise mit 5 bis 30, bevorzugt mit 7 bis 20 und besonders bevorzugt mit 7 bis 10 Kohlenstoffatomen, insbesondere einen Rest, der sich von Methylcyclohexan, Ethylcyclohexan, Propylcyclohexan, n-Butylcyclohexan, Isobutylcyclohexan oder tert.- Butylcyclohexan ableitet;
- einen Alkyl-Aryl-Rest, der über ein aliphatisches oder ein aromatisches Kohlenstoffatom mit dem olefinisch ungesättigten Kohlenstoffatom verknüpft ist, vorzugsweise mit 7 bis 30, bevorzugt mit 7 bis 20 und besonders bevorzugt mit 7 bis 10 Kohlenstoffatomen, insbesondere einen Rest, der sich von Toluol, Xylol, Ethylbenzol, Propylbenzol, Isopropylbenzol, n-Butylbenzol, Isobutylbenzol oder tert.-Butylbenzol ableitet;
- einen Cyclolkyl-Aryl-Rest, der über ein cycloaliphatisches oder ein aromatisches Kohlenstoffatom mit dem olefinisch ungesättigten Kohlenstoffatom verknüpft ist, vorzugsweise mit 10 bis 30, bevorzugt mit 10 bis 20 und besonders bevorzugt mit 10 bis 16 Kohlenstoffatomen, insbesondere einen Rest, der sich von Cyclohexylbenzol ableitet; oder
- einen Alkyl-Cycloalkyl-Aryl-Rest, der über ein aliphatisches, ein cycloaliphatisches oder ein aromatisches Kohlenstoffatom mit dem olefinisch ungesättigten Kohlenstoffatom verknüpft ist, vorzugsweise mit 11 bis 30, bevorzugt 11 bis 20 und besonders bevorzugt 11 bis 16 Kohlenstoffatomen, insbesondere einen Rest, der sich von 1-Methyl-2-, -3- oder -4-cyclohexylbenzol ableitet.

Besonders bevorzugt wird die Veresterung mit mindestens einem Olefin der allgemeinen Formel 1 durchgeführt. Ganz besonders bevorzugt wird ein Olefin der allgemeinen Formel 1 verwendet, worin der Rest R³ ein Alkylrest ist.

Insbesondere wird die Veresterung mit Isobuten, durchgeführt. Insbesondere wird zu diesem Zweck das in der deutschen Patentanmeldung DE 100 36 958 3 A1, Spalte 2, Absatz [0011], bis Spalte 11, Absatz [0071], im Detail beschriebene Verfahren verwendet.

Demgemäß werden das erfindungsgemäße Verfahren und die erfindungsgemäße Verwendung bei (Meth)Acrylsäureestern verwendet, die als Hauptbestandteil tert.-Butyl(meth)acrylat enthalten oder die im Wesentlichen hieraus bestehen.

Bei dem erfindungsgemäßen Verfahren werden die Säuren durch filtrierende Adsorption abgetrennt. Nach Römpp Online 2007, »Filtrierende Adsorption«, wird hierunter eine Filtrationsmethode verstanden, bei der zwei chemisch verschiedene Stoffe aufgrund ihres unterschiedlichen Absorptionsvermögens voneinander getrennt werden, wenn man das zu trennende Gemisch in einem geeigneten Lösemittel löst und die Lösung durch ein Adsorbens filtriert, das nur eine Komponente zurückhält. Von der normalen Adsorption unterscheidet sich die filtrierende Adsorption durch ihre Selektivität, die durch die Auswahl eines selektiven Lösemittels und eines entsprechenden Adsorbens erreicht wird.

Hierbei erweist es sich als ein zusätzlicher Vorteil des erfindungsgemäßen Verfahrens, dass gegebenenfalls noch vorhandene Reste der zur Katalyse eingesetzten Säuren wie Schwefelsäure oder Sulfonsäuren mit abgetrennt werden.

Es ist außerdem ein ganz besonderer Vorteil des erfindungsgemäßen Verfahrens, dass für die filtrierende Adsorption gemäß der vorliegenden Erfindung kein zusätzliches Lösemittel notwendig ist, weil die für die vollständige oder nahezu vollständige Entfernung der Säuren notwendige hohe Selektivität überraschenderweise auch ohne zusätzliches Lösemittel erzielt wird.

Bei dem erfindungsgemäßen Verfahren wird mindestens ein pulverförmiges Adsorptionsmittel verwendet.

Das Adsorptionsmittel ist inert. Im Rahmen der vorliegenden Erfindung gilt ein Adsorptionsmittel als »inert«, wenn es zwar die Säuren absorbiert, indes unter den Bedingungen, unter denen das erfindungsgemäße Verfahren vorzugsweise durchgeführt wird (vgl. unten), auch nach längerer Kontaktzeit, vorzugsweise von mindestens einer Woche, weder mit den (Meth)Acrylsäureestern reagiert und/oder sich darin auflöst noch die Zersetzung und/oder die Polymerisation der Säuren und/oder der (Meth)Acrylsäureester initiiert und/oder katalysiert.

Vorzugsweise wird das Adsorptionsmittel aus der Gruppe, bestehend aus organischen basischen Anionenaustauschern, polymeren aminogruppenhaltigen Basen, Molekularsieben, anorganischen basischen Salzen, Metalloxiden und Metallhydroxiden, ausgewählt.

Geeignete organische basische Anionenaustauscher sind dreidimensional vernetzte Phenol-Formaldehydharze, Copolymerisate aus Styrol und Divinylbenzol oder Copolymerisate aus Methacrylaten und Divinylbenzol, die primäre, sekundäre, tertiäre und/oder quaternäre Ammoniumgruppen tragen (vgl. a. Römpp Online 2007, »Ionenaustauscher«). Geeignete organische basische Ionenaustauscher werden beispielsweise unter der Marke Amberlite^{®}, Amberlyst^{®} oder Ambersep^{®} von der Firma Rohm and Haas vertrieben.

Geeignete polymere aminogruppenhaltige Basen sind Polyethylenimine (vgl. a. Römpp Online 2007, »Polyethylenimine«) oder aminomethyliertes Polystyrol oder Polystyrole, die Phenylethyldiethylamin, Phenylethylmorpholin oder Phenylethylpiperidin tragen und die unter der Marke ScavengePore^{®} Resins von der Firma Rapp Polymere GmbH erhältlich sind.

Beispiele geeigneter Molekularsiebe sind Zeolithe (vgl. a. Römpp Online 2007, »Molekularsiebe«).

Ein Beispiel für ein geeignetes anorganisches basisches Salz ist Kaliumcarbonat.

Beispiele geeigneter Metalloxide sind aktive Aluminiumoxide, die durch Fällungsverfahren aus Aluminiumsalzlösungen oder durch Calcination aus alpha-Aluminiumhydroxyd bei niedrigen Temperaturen oder durch Stoßerhitzung hergestellt werden (vgl. a. Römpp Online 2007, »Aluminiumoxide«).

Ein Beispiel für ein geeignetes Metallhydroxid ist Calciumhydroxyd.

Insbesondere werden Kaliumcarbonat oder Calciumhydroxyd als Adsorptionsmittel in dem erfindungsgemäßen Verfahren verwendet.

Das Adsorptionsmittel kann auf einem Träger vorliegen, der im vorstehend genannten Sinne inert ist, der aber die zusätzliche Eigenschaft aufweist, dass er die Säuren nicht oder nur in sehr geringem Ausmaß adsorbiert und auch nicht von ihnen angegriffen wird.

Vorzugsweise wird der inerte Träger aus der Gruppe, bestehend aus Siliziumdioxiden und Silikaten ausgewählt. Insbesondere werden als Siliziumdioxide Kieselgele und als Silikate Kieselgure oder Diatomeenerden (vgl. a. Römpp Online, 2007, »Kieselgele« und »Kieselgur«) verwendet.

Das geträgerte und das ungeträgerte Adsorptionsmittel sind pulverförmig. Die Korngröße und die Korngrößenverteilung werden so gewählt, dass einerseits eine Oberfläche vorhanden ist, die für den raschen Kontakt zwischen Säure und Adsorptionsmittel und die quantitative Adsorption der Säuren groß genug ist, dass aber andererseits die leichte Filtrierbarkeit erhalten bleibt, d.h., dass sich beim Durchströmen des Pulvers kein zu hoher Druck aufbaut. Der Fachmann kann daher die geeigneten Korngrößen und Korngrößenverteilungen in einfacher Weise aufgrund seines allgemeinen Fachwissens gegebenenfalls unter Zuhilfenahme einiger weniger orientierender Versuche ermitteln.

Vorzugsweise haben die geträgerten und die ungeträgerten Adsorptionsmittel eine durch Siebanalyse ermittelte mittlere Korngröße von 50 bis 200 µm, bevorzugt 50 bis 150 µm und insbesondere etwa 80 bis 120 µm. Vorzugsweise ist die maximale Korngröße < 500 µm. Vorzugsweise enthalten die Adsorptionsmittel keinen oder nur einen geringen Feinstanteil mit Korngrößen < 5 µm.

Die Menge an Adsorptionsmittel, die pro Mengeneinheit an zu reinigenden (Meth)Acrylsäureestern eingesetzt werden, kann von Fall zu Fall sehr breit variieren und richtet sich in erster Linie nach dem Säuregehalt der (Meth)Acrylsäureester einerseits und nach der auf der Oberfläche der Adsorptionsmittel verfügbaren reaktiven basischen Zentren andererseits. Der Fachmann kann daher im Einzelfall die geeigneten Mengen an Adsorptionsmittel aufgrund seines allgemeinen Fachwissens gegebenenfalls unter Zuhilfenahme einiger weniger orientierender Versuche ermitteln.

Es ist ein weiterer besonderer Vorteil des erfindungsgemäßen Verfahrens, dass es problemlos im Labor-, Technikums- und Produktionsmaßstab durchgeführt werden kann. Das erfindungsgemäße Verfahren bietet keine methodischen Besonderheiten, sondern kann kontinuierlich oder diskontinuierlich in üblichen und bekannten, für die filtrierende Adsorption geeigneten Anlagen, wie Rührkessel, Säulen und Filtriereinheiten durchgeführt werden, wobei die üblichen und bekannten Sicherheitsmaßnahmen für die Handhabung von (Meth)Acrylsäure und (Meth)Acrylsäureestern eingesetzt werden.

Vorzugsweise liegt bei dem erfindungsgemäßen Verfahren das Adsorptionsmittel in der Form eines Schüttbetts vor, das von den säurehaltigen (Meth)Acrylsäureestern durchströmt wird. Vorzugsweise befindet sich das Schüttbett in einer Filtriereinheit. Insbesondere befindet sich das Schüttbett oder die Filtriereinheit in der Zuleitung von einem Vorratsgefäß für die die zu filtrierenden (Meth)Acrylsäureester, beispielsweise einem Vorratstank, zu einem Transportgefäß für die filtrierten (Meth)Acrylsäureester, beispielsweise einem Kesselwagen.

Vorzugsweise wird das erfindungsgemäße Verfahren bei Temperaturen zwischen 0 und 50°C, bevorzugt bei 10 bis 40°C und insbesondere bei 15 bis 35°C durchgeführt. Es kann unter normalem Luftdruck oder erhöhtem Druck, vorzugsweise einem Druck von bis zu 6 x 10⁶ Pa, durchgeführt werden.

Das erfindungsgemäße Verfahren liefert in einfacher und problemloser Weise (Meth)Acrylsäureester mit einem Säuregehalt < 100 ppm, vorzugsweise < 50 ppm. Es ist ein ganz besonderer Vorteil des erfindungsgemäßen Verfahrens, dass (Meth)Acrylsäureester, insbesondere tert.-Butyl(meth)acrylat, eines Säuregehalts, der unterhalb der Nachweisgrenze liegt, in einfacher Weise zugänglich sind.

### Beispiele

### Beispiele 1 bis 9

Die Abtrennung von Essigsäure, Propionsäure und Methacrylsäure aus tert.-Butylmethacrylat an Adsorber-Säulen

### Allgemeine Versuchsvorschrift:

Tert.-Butylmethacrylat (TBMA) wurde mit 150 ppm Essigsäure, 200 ppm Propionsäure und 150 ppm Methacrylsäure versetzt. HPLC-Säulen einer Länge von 125 mm und eines Durchmessers vor 4 mm wurden jeweils mit etwa 1 g eines der in der Tabelle 1 aufgeführten Adsorptionsmittel gefüllt. Das säurehaltige TBMA wurde mit einer HPLC-Pumpe mit einem konstanten Fluss von jeweils 2 ml/min über die HPLC-Säule gepumpt. Der Druck jeweils lag bei 1,7 bis 1,8 x 10⁶ Pa.

Bei jeder Säule wurden im Abstand von 15 bis 30 min die Fraktionen gewechselt. Es wurden jeweils 2 g der Fraktionen mit jeweils 4 g TRIS-Lösung [300 ppm TRIS (= Tris(hydroxymethyl)aminomethan) in destilliertem Wasser] versetzt und während 60s intensiv geschüttelt. Nach der Phasentrennung wurde von den wässrigen Phasen jeweils eine Probe entnommen. Der Säuregehalt der Proben wurde mittels Ionenchromatographie mit einem Metrohm^{®} Ionenchromatograph (inklusive Pumpe, Wechsler und Auswerte-PC; Säule: Hamilton PRP X100; Laufmittel: 1 mmol/l Phthalsäure in Wasser/Methanol 9:1, mit TRIS auf einen pH-Wert von 4,3 eingestellt; injiziertes Volumen: 10 µl; Durchfluss: 1,5 ml/min).

Die Güte der Säureabtrennung wurde danach beurteilt, wie lange die Säuren in den jeweiligen Säulen zurückgehalten wurden, und im Vergleich qualitativ wie folgt benotet: "+" = befriedigend; " + +" = gut; "+ + + "= sehr gut. Die Tabelle 1 gibt einen Überblick über die verwendeten Adsorptionsmittel und ihre Benotung.

**Tabelle 1: Verwendete Adsorptionsmittel und Güte der Säureabtrennung**

| Beispiel | Adsorptionsmittel | Note |
|---|---|---|
| 1 | Amberlite^{®} A21^{a)} | + + |
| 2 | Amberlite^{®} IRA 96^{a)} getrocknet | + |
| 3 | Amberlyst^{®} IRA 67^{a)} getrocknet | + |
| 4 | Ambersep^{®} 900 OH^{a)} getrocknet | + + |
| 5 | Calciumhydroxyd-Pulver | + + +^{b)} |
| 6 | Kaliumcarbonat-Pulver | + + +^{c)} |
| 7 | Alox 90 aktiv neutral^{d)} | + + |
| 8 | Molekularsieb 5 Angström | + |
| 9 | ScavengePore^{®} Phenylethylpiperidine) | + |

| | | |
|---|---|---|
| a) Organische basische Anionenaustauscher der Firma Rohm and Haas; b) mittlere Korngröße (Siebanalyse): 100 µm; keine Säuren mehr nachweisbar; 0,84g Calciumhydroxyd reichten theoretisch für 3,6 kg TBMA; c) mittlere Korngröße (Siebanalyse): 100 µm; keine Säure mehr nachweisbar; 1,52g Kaliumcarbonat reichten theoretisch für 4,41 kg TBMA; e) aktives Aluminiumoxid; d) Phenylethylpiperidin-Gruppen enthaltendes Polystyrol der Firma Rapp Polymere GmbH; | | |

Pulverförmiges Calciumhydroxyd und Kaliumcarbonat einer mittleren Korngröße (Siebanalyse) von jeweils 100 µm erwiesen sich als die wirksamsten Adsorptionsmittel.

### Beispiele 10

Die Abtrennung von Acrylsäure aus Acrylsäureestern und von Methacrylsäure aus Methacrylsäureestern

### Allgemeine Versuchsvorschrift:

Der Gehalt von tert.-Butylacrylat (TBA), TBMA, n-Butylacrylat (NBA), Methylacrylat (MA) und Methylmethacrylat (MMA) an Acrylsäure (AS) bzw. Methacrylsäure (MAS) wurde durch potentiometrische Titration bestimmt. Jeweils 200 ml der genannten Ester wurden jeweils mit 0,5 g, 5 g und 20 g Kaliumcarbonat-Pulver einer mittleren Korngröße (Siebanalyse) von 100 µm versetzt und während 2 Stunden gerührt. Anschließend wurde das Kaliumcarbonat abfiltriert, und es wurde der Säuregehalt der Filtrate durch potentiometrische Titration bestimmt. Die Versuchsergebnisse finden sich in der Tabelle 2. Sie zeigen erneut die besonders hohe Wirksamkeit des Kaliumcarbonat-Pulvers.

**Tabelle 2: Beispiel 10 - Versuchsergebnisse**

| (Meth)Acrylsäureester: | TBA | TBMA | NBA | MA | MMA |
|---|---|---|---|---|---|
| Säuregehalt (ppm): | | | | | |
| Unbehandelt: | 514 (AS) | 206 (MAS) | 47 (AS) | 15 (AS) | 12 (MAS) |
| 0,5 g Kaliumcarbonat: | 404 | 170 | 37 | 15 | 12 |
| 5 g h Kaliumcarbonat: | 135 | 63 | 10 | 10 | 10 |
| 20 g Kaliumcarbonat: | 14 | 14 | < 10 | < 10 | < 10 |

### Beispiel 12

Die Abtrennung von Säuren aus TBMA mit Kaliumcarbonat im Produktionsmaßstab

Für Beispiel 12 wurde TBMA eines Säuregehalts von 500 ppm verwendet. Ein Tankwagen eines Volumens von 50 t wurde mit 17 m³/h TBMA befüllt. Dabei wurde das TBMA durch eine Filtriereinheit geleitet, die 235 kg Kaliumcarbonat-Pulver einer mittleren Korngröße (Siebanalyse) von 100 µm enthielt. Die resultierende Tankfüllung wies einen Säuregehalt < 50 ppm auf.

## Patentansprüche

1. Verfahren zum Entfernen von Säuren aus (Meth)Acrylsäureestern, **dadurch gekennzeichnet, dass** die Säuren durch filtrierende Adsorption an mindestens einem pulverförmigen Adsorptionsmittel von den (Meth)Acrylsäureestern abgetrennt werden.

2. Verfahren nach Anspruch 1, **dadurch gekennzeichnet, dass** das Adsorptionsmittel aus der Gruppe, bestehend aus organischen basischen Anionenaustauschern, polymeren aminogruppenhaltigen Basen, Molekularsieben, anorganischen basischen Salzen, Metalloxiden und Metallhydroxiden, ausgewählt wird.

3. Verfahren nach Anspruch 2, **dadurch gekennzeichnet, dass** das Adsorptionsmittel aus der Gruppe, bestehend aus anorganischen basischen Salzen und Metallhydroxiden, ausgewählt wird.

4. Verfahren nach Anspruch 3, **dadurch gekennzeichnet, dass** als anorganisches basisches Salz Kaliumcarbonat und als Metallhydroxid Calciumhydroxyd verwendet werden.

5. Verfahren nach einem der Ansprüche 1 bis 4, **dadurch gekennzeichnet, dass** das Adsorptionsmittel auf einem inerten Träger vorliegt.

6. Verfahren nach Anspruch 5, **dadurch gekennzeichnet, dass** der inerte Träger aus der Gruppe, bestehend aus Siliziumdioxiden und Silikaten, ausgewählt wird.

7. Verfahren nach Anspruch 6, **dadurch gekennzeichnet, dass** als Siliziumdioxide Kieselgele und als Silikate Kieselgure oder Diatomeenerden verwendet werden.

8. Verfahren nach einem der Ansprüche 1 bis 7, **dadurch gekennzeichnet, dass** die geträgerten und die ungeträgerten Adsorptionsmittel eine durch Siebanalyse ermittelte mittlere Korngröße von 50 bis 200 µm haben.

9. Verfahren nach Anspruch 8, **dadurch gekennzeichnet, dass** die mittlere Korngröße bei 50 bis 150 µm liegt.

10. Verfahren nach einem der Ansprüche 1 bis 9, **dadurch gekennzeichnet, dass** die Säuren aus der Gruppe, bestehend aus (Meth)Acrylsäure, Diacrylsäure und gesättigten organischen Säuren, abgetrennt werden.

11. Verfahren nach Anspruch 10, **dadurch gekennzeichnet, dass** als gesättigte organische Säuren Alkanmonocarbonsäuren mit 1 bis 4 Kohlenstoffatome im Alkylrest abgetrennt werden.

12. Verfahren nach Anspruch 11, **dadurch gekennzeichnet, dass** Alkanmonocarbonsäuren aus der Gruppe, bestehend aus Ameisensäure, Essigsäure und Propionsäure, abgetrennt werden.

13. Verfahren nach einem der Ansprüche 1 bis 12, **dadurch gekennzeichnet, dass** die (Meth)Acrylsäureester durch die säurekatalysierte Veresterung von (Meth)Acrylsäure hergestellt werden.

14. Verfahren nach Anspruch 13, **dadurch gekennzeichnet, dass** (Meth)Acrylsäure mit mindestens einer Verbindung, ausgewählt aus der Gruppe, bestehend aus aliphatischen Monoalkoholen, cycloaliphatischen Monoalkoholen, aliphatisch-cycloaliphatischen Monoalkoholen, worin die Hydroxylgruppe mit einem aliphatischen oder einem cycloaliphatischen Kohlenstoffatom verknüpft ist, aliphatisch-aromatischen Monoalkoholen, worin die Hydroxylgruppe mit einem aliphatischen Kohlenstoffatom verknüpft ist, cycloaliphatisch-aromatischen Monoalkoholen, worin die Hydroxylgruppe mit einem cycloaliphatischen Kohlenstoffatom verknüpft ist, aliphatisch-cycloaliphatischen-aromatischen Monoalkoholen, worin die Hydroxylgruppe mit einem aliphatischen oder einem cycloaliphatischen Kohlenstoffatom verknüpft ist, und Olefinen der allgemeinen Formel I: 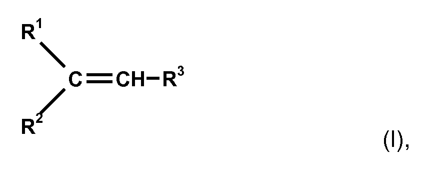 worin R¹ und R² gleich oder voneinander verschieden sein können und für einen Alkyl-Rest stehen und R³ für ein Wasserstoffatom, einen Alkylrest, einen

15. Verfahren nach Anspruch 14, **dadurch gekennzeichnet, dass** als Olefin der allgemeinen Formel I Isobuten verwendet wird.

16. Verfahren nach einem der Ansprüche 1 bis 15, **dadurch gekennzeichnet, dass** die (Meth)Acrylsäureester nach der Abtrennung der Säuren einen Restsäuregehalt < 100 ppm aufweisen.

17. Verfahren nach Anspruch 13, **dadurch gekennzeichnet, dass** der Restsäuregehalt < 50 ppm ist.

18. Verfahren nach einem der Ansprüche 1 bis 17, **dadurch gekennzeichnet, dass** sich das Adsorptionsmittel in einem Schüttbett befindet, dass von den säurehaltigen (Meth)Acrylsäureestern durchströmt wird.

19. Verfahren nach Anspruch 18, **dadurch gekennzeichnet, dass** sich das Schüttbett in einer Filtriereinheit befindet.

20. Verfahren nach Anspruch 18 oder 19, **dadurch gekennzeichnet, dass** sich das Schüttbett oder die Filtriereinheit in der Zuleitung von einem Vorratsgefäß für die zu filtrierenden (Meth)Acrylsäureester zu einem Transportgefäß für die filtrierten (Meth)Acrylsäureester befindet.

21. Verwendung von pulverförmigen Materialien, die mindestens einen Stoff, ausgewählt aus der Gruppe, bestehend aus organischen basischen Anionenaustauschern, polymeren aminogruppenhaltigen Basen, Molekularsieben, anorganischen basischen Salzen, Metalloxiden und Metallhydroxiden, enthalten oder hieraus bestehen als filtrierende Adsorptionsmittel zur Entfernung von Säuren aus (Meth)Acrylsäureestern.

22. Verwendung nach Anspruch 21, **dadurch gekennzeichnet, dass** die pulverförmigen Materialien aus der Gruppe, bestehend aus anorganischen basischen Salzen und Metallhydroxiden, ausgewählt werden.

23. Verwendung nach Anspruch 22, **dadurch gekennzeichnet, dass** als Metallhydroxid Calciumhydroxyd und als basisches Salz Kaliumcarbonat verwendet werden.

24. Verwendung nach einem der Ansprüche 21 bis 23, **dadurch gekennzeichnet, dass** das Adsorptionsmittel auf einem inerten Träger vorliegt.

25. Verwendung nach Anspruch 24, **dadurch gekennzeichnet, dass** der inerte Träger aus der Gruppe, bestehend aus Siliziumdioxiden und Silikaten, ausgewählt wird.

26. Verwendung nach Anspruch 25, **dadurch gekennzeichnet, dass** als Siliziumdioxide Kieselgele und als Silikate Kieselgure oder Diatomeenerden verwendet werden.

27. Verwendung nach einem der Ansprüche 21 bis 26, **dadurch gekennzeichnet, dass** die geträgerten und die ungeträgerten Adsorptionsmittel eine durch Siebanalyse ermittelte mittlere Korngröße von 50 bis 200 µm haben.

28. Verfahren nach Anspruch 27, **dadurch gekennzeichnet, dass** die mittlere Korngröße bei 50 bis 150 µm liegt.
